Europäisches Patentamt

European Patent Office

Office européen des brevets

Publication number: **0 289 636**
A1

## EUROPEAN PATENT APPLICATION

(21) Application number: 87106557.9

(51) Int. Cl.⁴ **A61K 9/10** , **A61K 31/56**

(22) Date of filing: 06.05.87

(43) Date of publication of application:
09.11.88 Bulletin 88/45

(84) Designated Contracting States:
CH DE FR GB IT LI

(71) Applicant: ASAHI DENKA KOGYO KABUSHIKI
KAISHA
2-35, 7-chome Higashiogu
Arakawa-ku Tokyo 116(JP)

Applicant: Ajinomoto Co., Ltd.
5-8, 1-chome
Kyobashi Chuo-ku Tokyo(JP)

(72) Inventor: Shirakawa, Yoichi
Asahi Denka Kogyo K.K. 2-35, 7-chome,
Higashiogu
Arakawa-ku Tokyo(JP)
Inventor: Motohashi, Tatsurou
c/o Ajinomoto Co., Inc. 5-8, 1-chome,
Kyobashi
Chuo-ku Tokyo(JP)

(74) Representative: Patentanwälte Grünecker,
Kinkeldey, Stockmair & Partner
Maximilianstrasse 58
D-8000 München 22(DE)

(54) Emulsified or solubilized sterol composition.

(57) An emulsified or solubilized sterol composition, wherein said sterol(s) are emulsified or solubilized as such in an aqueous solution of polyhydroxy compound(s) containing sucrose fatty acid ester(s) and/or polyglycerol fatty acid ester(s) or liquid polyhydroxy compound(s) without using any solvent such as fat or oil is disclosed. This composition exhibits a considerably high shelf stability and is extremely useful in various products including food, cosmetics, drugs and agricultural chemicals.

EP 0 289 636 A1

## EMULSIFIED OR SOLUBILIZED STEROL COMPOSITION

BACKGROUND OF THE INVENTION

Field of the Invention

This invention relates to an emulsified or solubilized sterol composition. More particularly, it relates to an emulsified or solubilized sterol composition which has a considerably high shelf stability. The emulsified or solubilized sterol composition of the present invention is extremely useful for various products including food, cosmetics, drugs and agricultural chemicals.

Description of the Prior Art:

Recently the physiological and pharmacological properties of sterols, which are obtained from unsaponifiable fractions formed in the saponification of animal and vegetable fats and oils, have attracted public attention.

A sterol is a high-melting compound which is hardly soluble in water and has a melting point of approximately 150°C. Although it is possible to emulsify a sterol in an aqueous phase by dissolving the same in, for example, a fat or oil, the poor compatibility thereof with fats or oils makes it difficult to obtain a stably emulsified or solubilized composition, which brings about a serious problem in the application of the same as a liquid preparation.

When a sterol is to be used in, for example, food, cosmetics or drugs, it is desirable to emulsify or solubilize the same with the use of highly safe compound(s). However there has been proposed no composition wherein sterol(s) are emulsified or solubilized with the use of such highly safe compound(s) as described above.

SUMMARY OF THE INVENTION

It is an object of the present invention to provide a composition wherein sterol(s) per se are emulsified or solubilized without using any fats or oils as a solvent for the sterol(s).

Further it is another object of the present invention to provide an emulsified or solubilized sterol composition having a considerably high shelf stability.

Furthermore it is another object of the present invention to provide an emulsified or solubilized sterol composition which is highly suitable for various products including food, cosmetics, drugs and agricultural chemcals.

According to the present invention, the objects as mentioned above can be achieved with the preparation of an emulsified or solubilized sterol composition wherein sterol(s) are emulsified or solubilized in an aqueous solution of polyhydroxy compound(s) or in liquid polyhydroxy compound(s) containing sucrose fatty acid ester(s) and/or polyglycerol fatty acid ester(s).

DETAILED DESCRIPTION OF THE INVENTION

Examples of the sterols to be used in the present invention are 5,7,22-cholestatrienol, 7-dehydrocholesterol, 22-dehydrocholesterol, 24-dehydrocholesterol, zymosterol, cholesterol, $\Delta^7$-cholesterol, 7-coprostenol, cholestanol, coprostanol, epicoprostanol, cerebrosterol, 22-$\alpha$-oxycholesterol, 22-dihydroerogosterol, 7,24(28)-erogostadienol, campesterol, neospongosterol, 7-ergostenol, cerebisterol, cor-bisterol, stigmasterol, focosterol, $\alpha$-spinasterol, sargasterol, 7-dehydrocryonasterol, poriferasterol, chondril-lasterol, $\beta$-sitosterol, cryonasterol ($\gamma$-sitosterol), 7-stigmastenol, 22-stigmastenol, dihydro-$\gamma$-sitosterol, $\beta$-

2

sitostanol, 14-dehydroergosterol, 24(28)-dehydroergosterol, ergosterol, brassicasterol, 24-methylenecholesterol, ascosterol, episterol, fecosterol and 5-dihydroergosterol.

Preferable examples of the sucrose fatty acid esters are mono-or diesters of sucrose and saturated and/or unsaturated fatty acids having 10 to 24 carbon atoms and mixtures thereof. Examples of the fatty acids constituting these sucrose fatty acid esters include caprylic, capric, lauric, myristic, palmitic, stearic, arachic, behenic, zoomaric, oleic, elaidic, erucic, linoleic, linolenic and arachidonic acids. Examples of the abovementioned sucrose fatty acid esters are sucrose monoesters such as sucrose monopalmitate, sucrose monostearate and sucrose monooleate; sucrose diesters such as sucrose dipalmitate, sucrose distearate and sucrose dioleate; and mixtures thereof. Among these sucrose fatty acid esters, those having an HLB of at least 9 and containing at least 50% by weight of monoester(s) are particularly preferable.

Preferable examples of the polyglycerol fatty acid esters to be used in the present invention are mono-, di-or polyesters of polyglycerol(s) and saturated and/or unsaturated fatty acid(s) having 10 to 24 carbon atoms and mixtures thereof. Examples of the fatty acids constituting these polyglycerol fatty acid esters are those as cited above in regard to the sucrose fatty acid esters. It is preferable the polyglycerol has the degree of polymerization of about 4 to about 10. Examples of the polglycerol are decaglycerol, hexaglycerol and tetraglycerol. Among these polyglycerol fatty acid esters, those which comprise the abovementioned polyglycerol(s) and fatty acid(s) having 16 to 18 carbon atoms, such as stearic, oleic and palmitic acid(s), contain at least 50% by weight of monoester(s) and have an HLB of at least 8 are particularly pre ferable.

The composition of the present invention may contain the sucrose fatty acid ester(s) and/or polyglycerol fatty acid ester(s) in an amount of 0.5 to 10 parts by weight, preferably 1.0 to 5 parts by weight, per part by weight of the sterol(s). In order to completely solubilized the sterol(s), the composition preferably contains the same in an amount of 1.5 parts by weight or more.

The polyhydroxy compounds to be used in the present invention are not strictly limited so long as they have at least two hydroxyl groups. Examples thereof include dihydric and trihydric alcohols such as ethylene glycol, propylene glycol and glycerol; sugar alcohols such as sorbitol and mannitol; sugars such as glucose, galactose, mannose, fructose, xylose, arabinose, saccharose, maltose and lactose; and various invert sugars (starch sugars), isomerized sugars, dextrins and syrups obtained by hydrolyzing starch syrup, glucose or honey. Examples of the liquid polyhydroxy compounds are those which are selected from among the polyhydroxy compounds as described above and in the form of a liquid at room temperature, e.g., di-or trihydric alcohols such as propylene glycol, ethylene glycol or glycerol. Among these compounds, glycerol is particularly preferable.

The liquid polyhydroxy compound(s) which can be used range from aqueous solution(s) thereof of a concentration of approximately 30% by weight to the one which is in a substantially absolute state. However it is preferable that these compounds contain at least a small amount of water in order to achieve more stable emulsification or solubilization.

Thus it is preferable in the present invention that the polyhydroxy compound(s) are used in the form of an aqueous solution of a concentration of 50 to 90% by weight, regardless of its form, i.e., solid or liquid.

The composition of the present invention should contain at least 5 parts by weight, preferably 10 to 100 parts by weight, of the polyhydroxy compound(s) per part by weight of the sterol(s).

The composition of the present invention may be prepared by, for example, adding said sucrose fatty acid ester(s) and/or polyglycerol fatty acid ester(s) to the aqueous solution of said polyhydroxy compound-(s) or liquid polyhydroxy compound(s); heating the obtained mixture to 50 to 60°C to thereby completely dissolve the additive(s); adding powdery sterol(s) thereto; stirring the obtained mixture at 50 to 90°C to thereby dissolve the sterol(s); and appropriately diluting the obtained solution if required. In this process, it is required to add the sucrose fatty acid ester(s) and/or polyglycerol fatty acid ester(s) in an amount within the range as determined above to the sterol(s) to be emulsified or solubilized; and to use the aqueous solution of the polyhydroxy compound(s) or liquid polyhydroxy compound(s) in a sufficient amount and at a sufficient concentration to completely dissolve said sucrose fatty acid ester(s) and/or polyglycerol fatty acid ester(s).

Since the composition of the present invention comprises sterol(s), which are solid at room temperature, very stably emulsified or solubilized without using any toxic compounds, it is extremely suitable for various products including food, cosmetics, drugs and agricultural chemicals.

The composition of the present invention may be used at various concentrations depending on the purpose. When diluted to various extents, it shows stable emulsification or solubilization without causing any separation of the sterol(s). Thus the composition of the present invention is considerably excellent in the emulsification or solubilization properties.

To further illustrate the present invention, and not by way of limitation, the following Examples will be given. The componets of each sterol composition used therein are as follows.

3

| Component (% by weight) | Composition No. | | | | |
|---|---|---|---|---|---|
| | 1 | 2 | 3 | 4 | 5 |
| brassicasterol | 7 | 0.5 | – | 1 | 0.5 |
| campesterol | 28 | 6 | – | 15 | 70 |
| stigmasterol | 15 | 3 | – | 80 | 24 |
| β-sitosterol | 50 | 10.5 | – | 4 | 5.5 |
| cholesterol | – | 80 | 100 | – | – |

(Note) unit: % by weight

Example 1

50 parts by weight of glycerol and 10 parts by weight of water were introduced into a vessel equipped with a stirrer and 4.4 parts by weight of a sucrose fatty acid ester (Ryoto Sugar Ester P-1570 mfd. by Ryoto Co., Ltd.; containing 70% by weight of monoesters, having an HLB of 15 and comprising 30% of stearic acid and 70% of palmitic acid as the fatty acid components) was added thereto. The obtained mixture was stirred for 20 minutes at 60°C. After confirming that the sucrose fatty acid ester was completely dissolved, two parts by weight portions of the sterol compositions No. 1 to No. 5 as shown in the above Table were added thereto as such, i.e., in the form of a powder. Each mixture thus obtained was stirred at 80°C until crystals of the phytosterols were dissolved. Thus it was diluted with 33.6 parts by weight of water and cooled to room temperature. Thus a translucent emulsified or solubilized sterol composition was obtained. When diluted 200-fold with water, each composition showed somewhat blue coloration and stable emulsification or solubilization.

Example 2

50 parts by weight of glycerol and 20 parts by weight of water are introduced into a vessel equipped with a stirrer and four parts by weight of a sucrose fatty acid ester (Ryoto Sugar Ester P-1670 mfd. by Ryoto Co., Ltd.; containing 75% by weight of monoesters, having an HLB of 16 and comprising 30% of stearic acid and 70% of palmitic acid as the fatty acid components) was added thereto. The obtained mixture was heated to 60°C. After confirming that the sucrose fatty acid ester was completely dissolved, two parts by weight portions of the sterol compositions No. 1 to No. 5 as shown in the above Table were added thereto as such, i.e., in the form of a powder. Each mixture thus obtained was stirred at 80°C until crystals of the phytosterols were dissolved. After adding water to prepare 100 parts by weight of a solution, it was cooled to room temperature. Thus a translucent emulsified or solubilized sterol composition containing 2% by weight of the sterols was obtained. When diluted 500-fold with water, each composition showed somewhat blue coloration and stable emulsification or solubilization.

Example 3

The procedure of Example 1 was followed except that the sucrose fatty acid ester was replaced with decaglycerol monooleate (SY Glyster MO-750 mfd. by Sakamoto Pharmacutical Co., Ltd.; HLB 15) to thereby give translucent emulsified or solubilized sterol compositions. When diluted 200-fold with water, each composition showed somewhat blue coloration and stable emulsification or solubilization.

Example 4

The procedure of Example 1 was followed except that 50 parts by weight of glycerol and 10 parts by weight of water were replaced with 60 parts by weight of a 70% aqueous solution of sorbitol to thereby give white emulsified or solubilized sterol compositions. When diluted 200-fold with water, each composition showed white coloration and stable emulsification or solubilization.

Example 5

The procedure of Example 1 was followed except that 50 parts by weight of glycerol and 10 parts by weight of water were replaced with 45 parts by weight of an isomerized sugar of a solid content of 75% and 15 fied or solubilized sterol compositions. When diluted 200-fold with water, each composition showed white coloration and stable emulsification and solubilization.

## Claims

1. An emulsified or solubilized sterol composition wherein said sterol(s) are emulsified or solubilized in an aqueous solution of polyhydroxy compound(s) or in liquid polyhydroxy compound(s) containing sucrose fatty acid ester(s) and/or polyglycerol fatty acid ester(s).

2. An emulsified or solubilized sterol composition as set forth in Claim 1, wherein said sucrose fatty acid ester(s) and/or polyglycerol fatty acid ester(s) are employed in an amount of 0.5 to 10 parts by weight per part by weight of said sterol(s).

3. An emulsified or solubilized sterol composition as set forth in Claim 1, wherein said poly hydroxy compound(s) and/or liquid polyhydroxy compound(s) are employed in an amount of 5 to 100 parts by weight per part by weight of said sterol(s).

4. An emulsified or solubilized sterol composition as set forth in Claim 1, wherein the concentration of said aqueous solution of polyhydroxy com pound(s) is 50 to 90% by weight.

5. An emulsified of solubilized sterol composition as set forth in Claim 1, wherein said liquid polyhydroxy compound(s) are employed in the form of an aqueous solution.

6. An emulsified of solubilized sterol composition as set forth in Claim 5, wherein the concentration of said liquid polyhydroxy compound(s) is 50 to 90% by weight.

7. An emulsified of solubilized sterol composition as set forth in Claim 1, wherein said sucrose fatty acid ester(s) comprises mono-or diester(s) of sucrose and saturated and/or unsaturated fatty acid(s) having 10 to 24 carbon atoms or a mixture thereof.

8. An emulsified of solubilized sterol composition as set forth in Claim 7, wherein said sucrose fatty acid ester(s) have an HLB of at least 9 and contain at least 50% by weight of monoester(s).

9. An emulsified of solubilized sterol composition as set forth in Claim 1, wherein said polyglycerol fatty acid ester(s) comprises mono-, di-or polyester(s) of polyglycerol and saturated and/or unsaturated fatty acid(s) having 10 to 24 carbon atoms or a mixture thereof.

10. An emulsified of solubilized sterol composition as set forth in Claim 9, wherein said polyglycerol fatty acid ester(s) have an HLB of at least 8 and contain at least 50% by weight of monoester(s).

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | GB-A-2 043 649 (MILES LABORATORIES INC.)<br>* Page 1, lines 21-27; page 3, lines 30-52 * | 1-10 | A 61 K 9/10<br>A 61 K 31/56 |
| Y | US-A-2 167 144 (MEAD JOHNSON & CO.)<br>* Page 1, left-hand column, lines 1-5,30-41; page 2, left-hand column, lines 36-39; claims 1,5,12 * | 1-10 | |
| A | US-A-3 495 011 (UNIMED INC.)<br>* Whole document * | 1-10 | |
| A | GB-A-2 166 107 (L'OREAL)<br>* Page 3, lines 24-60; page 5, example 1 * | 1-10 | |
| A | DE-A-1 617 557 (S. KAZUO)<br>* Page 19, lines 2-4; page 21, example 2,A; page 22, lines 1-6 * | 1-10 | TECHNICAL FIELDS SEARCHED (Int. Cl.4)<br><br>A 61 K |

The present search report has been drawn up for all claims

| Place of search | Date of completion of the search | Examiner |
|---|---|---|
| THE HAGUE | 12-11-1987 | FOERSTER W.K. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82